(19) Europäisches Patentamt / European Patent Office / Office européen des brevets

(11) **EP 1 031 373 B1**

(12) **EUROPÄISCHE PATENTSCHRIFT**

(45) Veröffentlichungstag und Bekanntmachung des Hinweises auf die Patenterteilung:
**22.09.2010 Patentblatt 2010/38**

(51) Int Cl.:
*B01J 8/02* *(2006.01)*    *C07C 29/151* *(2006.01)*
*C07C 29/154* *(2006.01)*    *F28D 7/16* *(2006.01)*
*F28F 9/02* *(2006.01)*

(21) Anmeldenummer: **00100525.5**

(22) Anmeldetag: **12.01.2000**

(54) **Reaktor zur katalytischen Umsetzung von Gasgemischen und Verfahren zur Benutzung des Reaktors**

Reactor and process for catalytic conversion of gaseous mixtures

Réacteur et procédé pour la conversion catalytique de mélanges gazeux

(84) Benannte Vertragsstaaten:
**AT BE CH CY DE DK ES FI FR GB GR IE IT LI LU MC NL PT SE**

(30) Priorität: **27.02.1999 DE 19908590**

(43) Veröffentlichungstag der Anmeldung:
**30.08.2000 Patentblatt 2000/35**

(73) Patentinhaber: **Lurgi GmbH**
**60439 Frankfurt am Main (DE)**

(72) Erfinder:
• **Brehm, Lothar**
**60598 Frankfurt am Main (DE)**
• **Göhna, Hermann**
**65812 Bad Soden (DE)**
• **Hohmann, Friedrich**
**63225 Langen (DE)**
• **Quass, Gunter**
**60435 Frankfurt am Main (DE)**

(74) Vertreter: **Lenz, Nanno Matthias**
**Keil & Schaafhausen,**
**Patentanwälte,**
**Cronstettenstrasse 66**
**60322 Frankfurt am Main (DE)**

(56) Entgegenhaltungen:
EP-A- 0 484 534    US-A- 2 989 383
US-A- 3 666 423    US-A- 5 827 901

• ASTANOVSKY K L ET AL: "REVOLUTIONARY REACTOR" N&M. NITROGEN AND METHANOL, GB,BRITISH SULPHUR PUBLISHING, LONDON, Nr. 232, 1. März 1998 (1998-03-01), Seiten 33-35,37-39, XP000740077 ISSN: 1462-2378

## Beschreibung

Die Erfindung betrifft einen Reaktor zur katalytischen Umsetzung eines Gasgemisches in einer Katalysatorschüttung, wobei der Reaktor zahlreiche Rohre zum Hindurchleiten eines Heiz- oder Kühlfluids aufweist, welche in der Katalysatorschüttung angeordnet sind und wobei die Eintrittsenden der Rohre mit mindestens einer Eintrittskammer und die Austrittsenden der Rohre mit mindestens einer Austrittskammer verbunden sind. Zur Erfindung gehören auch ein Verfahren und eine Anlage zur Methanol-Synthese mittels des Reaktors.

Reaktoren dieser Art sind bekannt und z. B. im US-Patent 5 827 901 beschrieben. Dieses Patent betrifft die Herstellung von Methanol, wobei neben einem wassergekühlten Röhrenreaktor auch ein gasgekühlter Reaktor mit einer Katalysatorschüttung verwendet wird. Die Kühlung der Schüttung erfolgt mittels Synthesegas, das durch die Rohre geleitet wird, die sich in der Schüttung befinden.

Aus der EP 0 484 534 A1 geht eine Vorrichtung zur Durchführung katalytischer Wirbelbettverfahren hervor, wobei insbesondere bei der Ausführungsform gemäß den Fig. 9 bis 11 in einem vertikalen zylindrischen Gehäuse entlang dessen Längsachse ein erster perforierter Ringsschuss und ein zweiter perforierter Ringschuss konzentrisch angeordnet sind. Zwischen der inneren Seitenfläche des Gehäuses und der äußeren Seitenfläche des perforierten Ringsschusses ist ein erster ringförmiger Raum gebildet. Zwischen der Innenfläche des ersten perforierten Ringsschusses und der Außenfläche des zweiten perforierten Ringsschusses ist ein zweiter ringförmiger Raum gebildet, in dem ein körniges Katalysatormaterial aufgenommen wird. Zwischen den Ringschüssen und ist eine Vielzahl von spiralförmig von dem inneren Ringschuss zu dem äußeren Ringschuss verlaufenden Wänden vorgesehen, zwischen denen Kanäle ausgebildet sind. Bei der Dampfkonvertierung von Kohlenoxid wird ein kohlenoxidhaltiges Dampf-Gas-Gemisch in den äußeren ringförmigen Raum eingeführt und strömt durch die Löcher des perforierten Ringsschusses radial in die Schicht des in den spiralförmigen Kanälen befindlichen Katalysators, folgt den Kanälen und tritt durch die Löcher in dem perforierten Ringschuss radial in den zylindrischen Hohlraum aus, aus welchem es nach unten aus dem Gehäuse abgeführt wird. Auf dem Katalysator läuft die Dampfkonvertierung des Kohlenoxids unter Wärmeentwicklung ab. Da der Katalysator sehr empfindlich gegen Temperaturüberhitzungen ist, soll der Prozess bei gleichbleibender Temperatur (isotherm) ablaufen. Daher muss die bei der Reaktion im Reaktor entstehende Reaktionswärme nach außen abgeführt werden. Hierzu werden die Wände durch vertikale Rohre gebildet, die mit vertikalen Streifen (Flossen) abwechseln, mit welchen sie über die gesamte Länge starr verbunden sind. Durch die Rohre kann ein Wärmeträger geführt werden, um die Reaktionswärme nach außen abzuführen. Kommt es dennoch zu einem Aufheizen der Reaktorkonstruktion, kann dies zu mechanischen Spannungen in der Vorrichtung und zu Beschädigungen des Reaktors führen.

Aufgabe der Erfindung ist es daher, eine Beschädigung des Reaktors durch Längenänderungen aufgrund von Temperaturschwankungen zu vermeiden.

Dies Aufgabe wird mit der Erfindung durch einen Reaktor mit den Merkmalen des Anspruchs 1, ein Verfahren mit den Merkmalen des Anspruchs 3 und eine Anlage mit den Merkmalen des Anspruchs 4 gelöst.

Hierdurch ergibt sich eine annähernd gleichmäßige Verteilung der Rohre in der Katalysatorschüttung und die gleichmäßige Kühlung dieser Schüttung. Gleichzeitig sollen die im Reaktor befindlichen Einbauten, durch die das Heiz- oder Kühlfluid geleitet wird, auf Temperaturänderungen dehnungsflexibel reagieren.

Beim Reaktor können die Verteilungskammern entweder nur von der Eintrittskammer oder nur von der Austrittskammer ausgehen, doch wird es zumeist zweckmäßig sein, wenn sowohl die Eintrittskammer als auch die Austrittskammer mit Verteilungskammern in gleicher Ausgestaltung versehen ist. Hierbei verlaufen die Rohre üblicherweise etwa senkrecht zwischen den Verteilungskammern. Auf diese Weise ergibt sich eine Konstruktion, die auch ganz oder teilweise außerhalb des Reaktors vorgefertigt werden kann.

Der Reaktor eignet sich für vielerlei Aufgaben zur katalytischen Umsetzung von Gasgemischen, wobei das Kühl- oder Heizfluid gasförmig oder flüssig sein kann. Eine Möglichkeit besteht darin, den Reaktor zur katalytischen Umsetzung eines Synthesegases, das vor allem aus den Komponenten $H_2$ und CO besteht, zum Erzeugen von Methanol zu verwenden. Hierbei herrscht im Reaktor ein Druck von üblicherweise 20 bis 120 bar, und die Temperaturen in der gasgekühlten Katalysatorschüttung liegen im Bereich von 180 bis 350°C. Einzelheiten der Methanolsynthese sind bekannt und z. B. im erwähnen US-Patent 5 827 901 beschrieben. Man leitet hierbei als Kühlgas ein $H_2$ und CO enthaltendes Synthesegas durch die Eintrittskammer, die mit der Eintrittskammer verbundenen Verteilungskammern, die mit den Verteilungskammern verbundenen Rohre, die mit der Austrittskammer verbundenen Verteilungskammern und zieht das Kühlgas durch die Austrittskammer ab, bevor man es zur Methanolsynthese über einen Kupferkatalysator leitet.

Ausgestaltungsmöglichkeiten des Reaktors und Einzelheiten zum Verfahren des Betriebs werden mit Hilfe der Zeichnung erläutert. Es zeigt:

Fig. 1     den Reaktor im Längsschnitt zusammen mit einem wassergekühlten Röhrenreaktor für die Methanolsynthese,

Fig. 2     einen Querschnitt durch den Reaktor nach der Linie II-II in Fig. 1,

Fig. 3     eine erste Variante benachbarter Rohre im Längsschnitt, in der Darstellung entsprechend Fig. 1 und

Fig. 4 eine zweite Variante der Anordnung benachbarter Rohre im Längsschnitt.

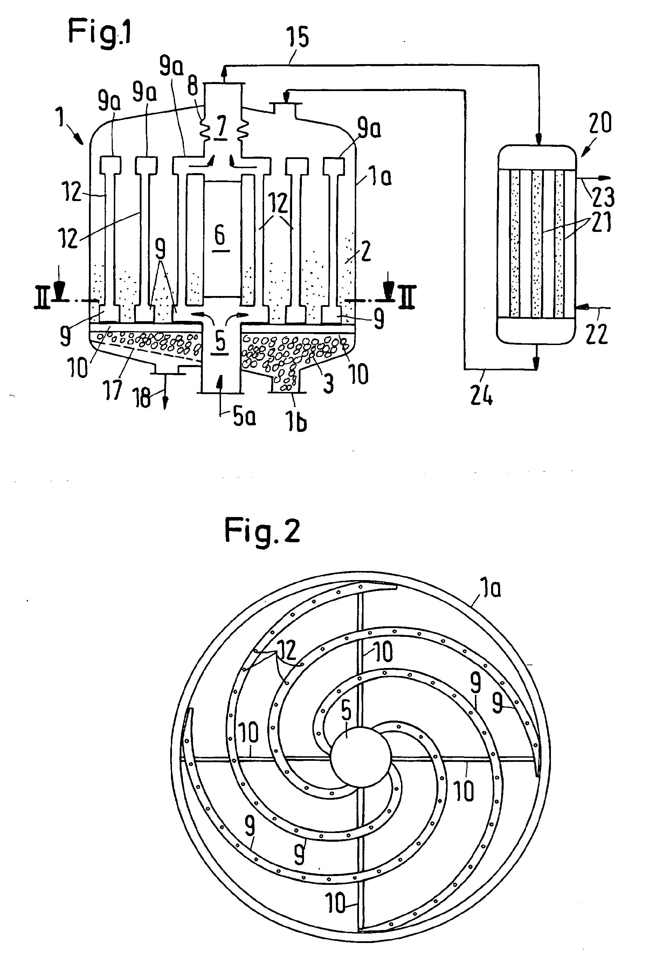

Der Reaktor (1) der Fig. 1 und 2 weist ein Gehäuse (1a) und darin eine Schüttung (2) eines körnigen Katalysators auf, die der besseren Übersichtlichkeit wegen nur teilweise dargestellt ist. Die Katalysatorschüttung (2) ruht auf einem Bett (3) aus Kugeln mit erheblich größerem Durchmesser als die Körnung der Katalysatorschüttung. Die Kugeln des Bettes (3) können z. B. aus Inertmaterial bestehen. Am unteren Ende des Gehäuses befindet sich ein Auslaß (1b) für die Kugeln und den Katalysator.

Zentral im Reaktor (1) sind eine Eintrittskammer (5), ein zylindrischer Verdrängerkörper (6) und eine Austrittskammer (7) übereinander angeordnet. Die Austrittskammer (7) ist mit einem Kompensator (8) zum Ausgleichen von Längenänderungen aufgrund von Temperaturänderungen versehen.

Von der Eintrittskammer (5) gehen mehrere Verteilungskammern (9) aus, die sich auf horizontalen Tragschienen (10) abstützen. Die Anzahl der Kammern (9) liegt üblicherweise zwischen 2 und 50. Jede Verteilungskammer (9) ist als gekrümmter Kanal ausgebildet, der zur Eintrittskammer hin offen und am anderen Ende geschlossen ist. Jede Verteilungskammer (9) hat etwa die Form einer Kreisevolvente, so daß sie mindestens 1,5 mal so lang ist wie der radiale Abstand zwischen der Eintrittskammer (5) und dem Reaktorgehäuse (1a).

Mathematisch kann die Kreisevolvente durch die bekannte Gleichung

$$S = 0,5 \bullet r \bullet x^2$$

beschrieben werden, dabei bedeuten:

    S = Bogenlänge der Evolvente,
    x = Drehwinkel im Bogenmaß,
    r = Radius der Eintritts- oder Austrittskammer.

Dieser idealen Evolvente muß die Form der Verteilungskammern (9) jedoch nur annähernd und keinesfalls genau folgen.

Von jeder Verteilungskammer (9) gehen mindestens zwanzig Rohre (12) aus, durch die das Kühl- oder Heizfluid geführt wird. Die Anzahl der Rohre, die mit einer Verteilungskammer (9) verbunden sind, kann beliebig größer als zwanzig sein und wird wohl üblicherweise nicht mehr als 300 betragen. Die Austrittskammer (7) ist ebenso wie die Eintrittskammer (5) mit Verteilungskammmern (9a) verbunden (vergleiche Fig. 1), und die Rohre (12) verlaufen zwischen den Verteilungskammern (9) und (9a). Kühl- oder Heizfluid, das in Richtung des Pfeils (5a) in die Eintrittskammer (5) einströmt, verteilt sich auf die Verteilungskammern (9), strömt durch die Rohre (12) zunächst in die Verteilungskammern (9a) und von da in die Austrittskammer (7), bis es durch die Leitung (15) abgezogen wird. Wenn es sich bei dem Kühlfluid um Methanol-Synthesegas handelt, wird dieses Synthesegas von der Leitung (15) z. B. in einen an sich bekannten Röhrenreaktor (20) geführt, der in Röhren (21) einen körnigen Kupferkatalysator enthält. Die Röhren (21) sind mit siedendem Wasser gekühlt, das Kühlwasser wird dem Reaktor (20) durch die Leitung (22) zugeführt, und ein Dampf-Wasser-Gemisch zieht man in der Leitung (23) ab.

Das Synthesegas, das im Reaktor (20) nur teilweise umgesetzt wird, verläßt den Reaktor (20) durch die Leitung (24) und wird in den Reaktor (1) und durch dessen Katalysatorschüttung (2) geleitet. Das Produktgemisch, das Methanoldampf und restliche Gase enthält, wird durch einen Rost (17) nach unten abgezogen und verläßt den Reaktor (1) durch die Leitung (18).

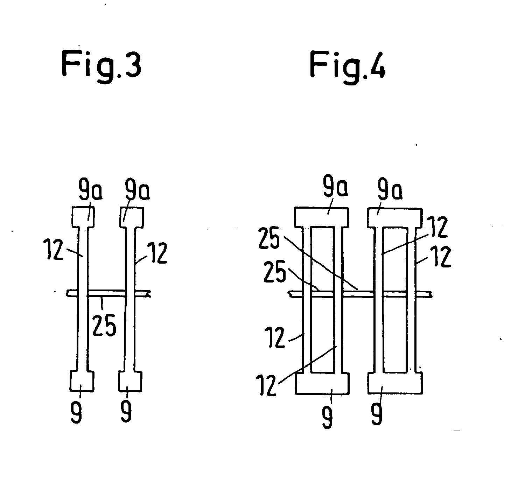

Fig. 3 zeigt im Längsschnitt zwei Rohre (12), die zwischen unteren Verteilungskammern (9) und oberen Verteilungskammern (9a) angeordnet sind. Zum Erhöhen der Stabilität der Anordnung dienen Streben (25), die mit den Rohren (12) verbunden und z. B. verschweißt sind. In Fig. 1 wurden diese Streben (25) der besseren Übersichtlichkeit wegen weggelassen.

Die Variante der Fig. 4 zeigt im Querschnitt untere Verteilungskammern (9) und obere Verteilungskammern (9a), die im Querschnitt größer als in Fig. 3 ausgebildet sind. Mit jeder Kammer (9) und (9a) sind zwei Rohre (12) verbunden und durch Streben (25) gegeneinander stabilisiert.

Beispiel

In einer Anlage gemäß Fig. 1 und 2 zur Erzeugung von 5000 t Methanol pro Tag wird folgendermaßen gearbeitet:

Der Reaktor (1) weist 17 Verteilungskammern (9) oder (9a) auf, wobei von jeder Kammer 180 vertikale Rohre (12) mit 25 mm Durchmesser und 10 m Länge ausgehen. Die Länge jeder Kammer beträgt 8 m. Der Reaktor (1) enthält 200 m$^3$ eines körnigen Cu-Zn-Katalysators, der aus 60 Gew.-% CuO, 30 Gew.-% ZnO und 10 Gew.-% $Al_2O_3$ besteht. Der mit Siedewasser gekühlte Röhrenreaktor (20) ist als zwei parallel betriebene Reaktoren ausgebildet, die insgesamt 11800 Rohre (21) von 45 mm Durchmesser und 8 m Länge enthalten, in die insgesamt 120 m$^3$ des zuvor genannten Katalysators eingefüllt sind.

In die Eintrittskammer (5) leitet man 75000 kmol/h eines Synthesegasgemisches, das aus 68,4 Vol.-% $H_2$, 8,4 Vol.-% CO, 8,3 Vol.-% $CO_2$ und Inerten besteht. Erwärmt auf 240°C tritt das Synthesegas mit einem Druck von 7,5 MPa in den Röhrenreaktor (20) ein. Das Gasgemisch der Leitung (24) hat eine Temperatur von 265°C und ist zusammengesetzt, wie in der folgenden Tabelle, Spalte A, angegeben:

Tabelle:

|  | A | B |
|---|---|---|
| CO (Vol.-%) | 4,5 | 2,1 |
| $H_2$ (Vol.-%) | 62,2 | 59,1 |
| $CO_2$ (Vol.-%) | 7,6 | 7,5 |
| Methanol (Vol.-%) | 7,4 | 11,1 |
| Wasserdampf (Vol.-%) | 1,9 | 2,7 |
| Methan (Vol.-%) | 16,1 | 17,2 |
| $N_2$ + Ar (Vol.-%) | 0,3 | 0,3 |

Aus dem Reaktor (1) zieht man durch die Leitung (18) ein Gemisch mit einer Temperatur von 220°C und der Zusammensetzung gemäß obiger Tabelle, Spalte B, ab, aus welchem man Methanol auskondensiert. Das verbleibende Gasgemisch, das $H_2$, CO und Methan enthält, wird bei der Erzeugung des Synthesegases mitverwendet.

**Patentansprüche**

1. Reaktor (1) zur katalytischen Synthese von Methanol bei einem Druck von 2 bis 12 MPa (20 bis 120 bar) und einer Temperatur von 180 bis 350 °C, in dem eine Katalysatorschüttung (2) und zahlreiche in der Katalysatorschüttung angeordnete Rohre (12) zur Durchleitung eines Kühlgases vorgesehen sind, wobei die Eintrittsenden der Rohre (12) mit einer Eintrittskammer (5) und die Austrittsenden der Rohre (12) mit einer Austrittskammer (7) verbunden sind, und
mit einer Leitung (24), über welche der Katalysatorschüttung (2) in dem Reaktor (1) teilweise umgesetztes Synthesegas oben derart zugeführt wird, dass es diese von oben nach unten durchströmt, wobei von der zylindrischen Eintrittskammer (5) und der zylindrischen Austrittskammer (7) zwei bis fünfzig Verteilungskammern (9, 9a) ausgehen, wobei mit jeder Verteilungskammer mindestens zwanzig Rohre (12) mit ihrem Eintrittsende oder Austrittsende verbunden sind, wobei die Verteilungskammern (9, 9a) als gekrümmte Kanäle ausgebildet sind, deren Mittellinie etwa die Form einer Kreisevolvente hat, wobei zentral im Reaktor (1) die Eintrittskammer (5), ein zylindrischer Verdrängerkörper (6) und die Austrittskammer (7) so übereinander angeordnet sind, dass die Eintrittskammer (5) unterhalb und die Austrittskammer (7) oberhalb des Verdrängungskörpers (6) liegen, und
wobei die Austrittskammer (7) mit einem Kompensator (8) zum Ausgleichen von Längenänderungen aufgrund von Temperaturänderungen versehen ist.

2. Reaktor nach Anspruch 1, **dadurch gekennzeichnet, dass** die Rohre (12) etwa senkrecht zu den Verteilungskammern (9, 9a) angeordnet sind.

3. Verfahren zur Methanolsynthese mittels eines ersten Reaktors (1) nach Anspruch 1 oder 2 und eines zweiten Reaktors (20),
wobei der erste Reaktor (1) eine Schüttung eines Kupferkatalysators und in der Schüttung zahlreiche Rohre (12) zum Hindurchleiten eines Kühlgases aufweist,
wobei die Rohre (12) mit Verteilungskammern (9, 9a) und die Verteilungskammern (9, 9a) mit einer von Kühlgas durchströmten Eintrittskammer (5) und einer Austrittskammer (7) verbunden sind,
wobei der zweite Reaktor (20) ein mit Siedewasser gekühlter Röhrenreaktor ist, der in Röhren (21) einen Kupferkatalysator enthält,
wobei man als Kühlgas ein $H_2$ und CO enthaltenes Synthesegas durch die Eintrittskammer (5), die mit der Eintrittskammer (5) verbundenen Verteilungskammern (9), die mit den Verteilungskammern (9, 9a) verbundenen Rohre (12), die mit der Austrittskammer (7) verbundenen Verteilungskammern (9a) und durch die Austrittskammer (7) leitet,
wobei man das Kühlgas anschließend in den zweiten Reaktor (20) leitet und dort teilweise umsetzt,
wobei man das teilweise umgesetzte Kühlgas zu dem ersten Reaktor (1) zurückführt und von oben nach unten durch die Katalysatorschüttung leitet,
wobei der Druck im ersten Reaktor (1) 2 bis 12 MPa (20 bis 120 bar) und die Temperatur in der Schüttung 180 bis 350 °C beträgt und
wobei man ein Produktgemisch unten aus dem ersten Reaktor (1) abzieht.

4. Anlage zur Durchführung eines Verfahrens nach Anspruch 3,
mit einem ersten Reaktor (1), in dem eine Schüttung (2) eines Kupferkatalysators und zahlreiche in der Katalysatorschüttung angeordnete Rohre (12) zur Durchleitung eines Kühlgases vorgesehen sind, wobei die Eintrittsenden der Rohre (12) mit einer Eintrittskammer (5) und die Austrittsenden der Rohre (12) mit einer Austrittskammer (7) verbunden sind, wobei von der Eintrittskammer (5) und der Austrittskammer (7) zwei bis fünfzig Verteilungskammern (9, 9a) ausgehen, wobei mit jeder Verteilungskammer mindestens zwanzig Rohre (12) mit ihrem Eintrittsende oder Austrittsende verbunden sind, wobei die Verteilungskammern (9, 9a) als gekrümmte Kanäle ausgebildet sind, deren Mittellinie etwa die Form einer Kreisevolvente hat,
wobei zentral im ersten Reaktor (1) die Eintrittskammer (3), ein zylindrischer Verdrängungskörper (6) und die Austrittskammer (7) so übereinander angeordnet sind, dass die Eintrittskammer (5) unterhalb und die Austrittskammer (7) oberhalb des Verdrängungskörpers (6) liegen,
wobei die Austrittskammer (7) mit einem Kompensator (8) zum Ausgleichen von Längenänderungen aufgrund von Temperaturänderungen versehen ist,
mit einem zweiten Reaktor (20), der in mit Siedewasser gekühlten Röhren (21) einen Kupferkatalysator enthält, wobei die Röhren (21) über eine Leitung (15) mit der Austrittskammer (7) des ersten Reaktors (1) verbunden sind, und
mit einer Leitung (24), über welche die Röhren (21) des zweiten Reaktors (20) mit dem ersten Reaktor (1) derart verbunden sind, dass das aus dem zweiten Reaktor (20) abgezogene teilweise umgesetzte Synthesegas der Katalysatorschüttung (2) in dem ersten Reaktor (1) oben zugeführt wird und diese von oben nach unten durchströmt.

**Claims**

1. A reactor (1) for the catalytic synthesis of methanol under a pressure from 2 to 12 MPa (20 to 120 bar) and at a temperature from 180 to 350°C in which a catalyst bed (2) and a multiplicity of pipes (12) arranged in the catalyst bed (2) are provided for feeding a cooling gas through, wherein the inlet ends of the pipes (12) communicate with an inlet chamber (5) and the outlet ends of the pipes (12) communicate with an outlet chamber (7), and
having a line (24) via which partially converted synthesis gas is fed upwards to the catalyst bed (2) in the reactor (1) in such manner that it passes from top to bottom through the catalyst bed
wherein two to fifty distribution chambers (9, 9a) lead from the cylindrical inlet chamber (5) and the cylindrical outlet chamber (7), wherein at least twenty pipes (12) are connected by their inlet end or outlet end to each distribution chamber, wherein the distribution chambers (9, 9a) are constructed as curved ducts, the midline of which is approximately in the form of an involute,
wherein the inlet chamber (5), a cylindrical central displacement element (6) and the outlet chamber (7) are arranged one above the other in the middle of the reactor (1) in such manner that the inlet chamber (5) is situated below the displacement element (6) and the outlet chamber (7) is situated above it, and wherein the outlet chamber (7) is equipped with a compensator (8) to compensate for changes in length brought about by changes in temperature.

2. The reactor as recited in claim 1, **characterized in that**
the pipes (12) are arranged approximately perpendicularly to the distribution chambers (9, 9a).

3. A process for synthesizing methanol using a first reactor (1) as recited in claim 1 or 2 and a second reactor (20),
wherein the first reactor (1) has a bed containing a copper catalyst, and a multiplicity of pipes (12) in the bed for transporting a cooling gas through it,
wherein the pipes (12) communicate with distribution chambers (9, 9a) and the distribution chambers (9, 9a) communicated with an inlet chamber (5) and an outlet chamber (7),
wherein the second reactor (20) is a tube reactor that is cooled with boiling water which contains a copper catalyst in tubes (21),
wherein a synthesis gas containing $H_2$ and CO as the cooling gas is passed through the inlet chamber (5), the distribution chambers (9) that communicate with the inlet chamber (5), the pipes (12) that communicate with the distribution chambers (9, 9a), the distribution chambers (9a) that communicate with the outlet chamber (7), and through the outlet chamber (7),
wherein the cooling gas is then fed into the second reactor (20) and partially converted there,
wherein the partially converted gas is returned to the first reactor (1) and passed through the catalyst bed from top to bottom, wherein the pressure in the first reactor (1) is 2 to 12 MPa (20 to 120 bar) and the temperature in the bed is 180 to 350°C, and wherein a product mixture is extracted from the bottom of the first reactor (1).

4. A plant for performing a process as recited in claim 3, having a first reactor (1) in which a bed (2) of a copper catalyst and a multiplicity of pipes (12) arranged in the catalyst bed are provided for transporting a cooling gas through the bed, wherein the inlet ends of the pipes (12) communicate with an inlet chamber (5) and the outlet ends of the pipes (12) communicate with an outlet chamber (7),
wherein two to fifty distribution chambers (9, 9a) lead from the inlet chamber (5) and the outlet chamber (7), wherein at least twenty pipes (12) are connected by their inlet end or outlet end to each distribution chamber, wherein the distribution chambers (9, 9a) are constructed as curved ducts, the midline of which is approximately in the form of an involute,
wherein the inlet chamber (3), a cylindrical displacement element (6) and the outlet chamber (7) are arranged one above the other in the centre of the first reactor (1) in such manner that the inlet chamber (5) is situated below the displacement element (6) and the outlet chamber (7) is situated above it,
wherein the outlet chamber (7) is equipped with a compensator (8) to compensate for changes in length caused by changes in temperature,
having a second reactor (20) that contains a copper catalyst in tubes (21) that are cooled with boiling water, wherein the tubes (21) are connected to the outlet chamber (7) of the first reactor (1) via a line (15), and having a line (24) via which the tubes (21) of the second reactor (20) are connected to the first reactor (1) in such manner that the partially converted synthesis gas extracted from the second reactor (20) is transported up to the catalyst bed (2) in the first reactor (1) flows through this reactor from top to bottom.

## Revendications

1. Réacteur (1) destiné à la synthèse catalytique de méthanol à une pression comprise entre 2 et 12 MPa (entre 20 et 120 bar) et à une température comprise entre 180 et 350 °C, dans lequel sont prévus un lit catalytique (2) et de nombreux tubes (12) disposés au sein dudit lit catalytique et destinés au passage d'un gaz réfrigérant, les extrémités d'entrée des tubes (12) communiquant avec une chambre d'entrée (5) et les extrémités de sortie des tubes (12) avec une chambre de sortie (7), et
pourvu d'un conduit (24) par lequel du gaz de synthèse ayant partiellement réagi est introduit par le haut dans le lit catalytique (2) au sein du réacteur (1), de façon à ce qu'il traverse le lit de haut en bas, la chambre d'entrée cylindrique (5) et la chambre de sortie cylindrique (7) étant chacune reliée à un nombre de chambres de distribution (9, 9a) compris entre deux et cinquante, chacune desdites chambres de distribution étant reliée à au moins vingt tubes (12), à savoir par l'extrémité d'entrée ou l'extrémité de sortie de ces derniers, les chambres de distribution (9, 9a) ayant la forme de canaux courbés dont la médiane possède approximativement la forme d'une développante de cercle,
la chambre d'entrée (5), un corps de déplacement cylindrique (6) et la chambre de sortie (7) étant disposés au centre du réacteur (1) de façon à ce que la chambre d'entrée (5) se trouve en-dessous et la chambre de sortie (7) se trouve en-dessus du corps de déplacement (6), et
la chambre de sortie (7) étant pourvue d'un compensateur (8) destiné à compenser les variations de longueur provoquées par les variations de température.

2. Réacteur selon la revendication 1, **caractérisé en ce que** les tubes (12) sont disposés globalement de façon perpendiculaire par rapport aux chambres de distribution (9, 9a).

3. Procédé de synthèse de méthanol au moyen d'un premier réacteur (1) selon les revendications 1 ou 2 et d'un deuxième réacteur (20),
le premier réacteur (1) comportant un lit d'un catalyseur de cuivre et, au sein dudit lit, de nombreux tubes (12) destinés au passage d'un gaz réfrigérant, les tubes (12) communiquant avec des chambres de distribution (9, 9a) et les chambres de distribution (9, 9a) avec une chambre d'entrée traversée (5) par un gaz réfrigérant et avec une chambre de sortie (7), le deuxième réacteur (20) étant un réacteur tubulaire refroidi à l'eau bouillante et contenant, dans des tubes (21), un catalyseur de cuivre,
du gaz de synthèse contenant du $H_2$ et du CO étant envoyé, en tant que gaz réfrigérant, à travers la chambre d'entrée (5), les chambres de distribution (9) communiquant avec la chambre d'entrée (5), les tubes (12) communiquant avec les chambres de distribution (9, 9a), les chambres de distribution (9a) communiquant avec la chambre de sortie (7) et à travers la chambre de sortie (7),
ledit gaz réfrigérant étant ensuite introduit dans le deuxième réacteur (20) où il subit une réaction partielle,
ledit gaz réfrigérant ayant partiellement réagi étant recyclé dans le premier réacteur (1) et envoyé, de haut en bas, à travers ledit lit catalytique, la pression dans le premier réacteur (1) étant comprise entre 2 et 12 MPa (20 à 120 bar) et la température dans ledit lit étant comprise entre 180 et 350 °C et un mélange de produits étant prélevé en bas du premier réacteur (1).

4. Installation destinée à la mise en oeuvre d'un procédé selon la revendication 3,
pourvue d'un premier réacteur (1), dans lequel sont prévus un lit (2) d'un catalyseur de cuivre et de nombreux tubes (12) disposés au sein dudit lit catalytique et destinés au passage d'un gaz réfrigérant, les extrémités d'entrée des tubes (12) communiquant avec une chambre d'entrée (5) et les extrémités de sortie des tubes (12) avec une chambre de sortie (7),
la chambre d'entrée cylindrique (5) et la chambre de sortie cylindrique (7) étant chacune reliée à un nombre de chambres de distribution (9, 9a) compris entre deux et cinquante, chacune desdites chambres de distribution étant reliée à au moins vingt tubes (12), à savoir par l'extrémité d'entrée ou l'extrémité de sortie de ces derniers, les chambres de distribution (9, 9a) ayant la forme de canaux courbés dont la médiane possède approximativement la forme d'une développante de cercle,
la chambre d'entrée (3), un corps de déplacement cylindrique (6) et la chambre de sortie (7) étant disposés au centre du premier réacteur (1) de façon à ce que la chambre d'entrée (5) se trouve en-dessous et la chambre de sortie (7) se trouve en-dessus du corps de déplacement (6),
la chambre de sortie (7) étant pourvue d'un compensateur (8) destiné à compenser les variations de longueur provoquées par les variations de température,
pourvue d'un deuxième réacteur (20) contenant, au sein de tubes (21) refroidis à l'eau bouillante, un catalyseur de cuivre, les tubes (21) communiquant à travers un conduit (15) avec la chambre de sortie (7) du premier réacteur (1), et pourvue d'un conduit (24) à travers lequel les tubes (21) du deuxième réacteur (20) communiquent avec le premier réacteur (1), de façon à ce que le gaz de synthèse ayant partiellement réagi, qui est prélevé dans le deuxième réacteur (20), soit introduit par le haut dans le lit catalytique (2) au sein du premier réacteur (1), de façon à ce qu'il traverse le lit de haut en bas.

Fig.1

Fig.2

# Fig.3    Fig.4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5827901 A

- EP 0484534 A1